# FASCICULE DE BREVET EUROPEEN

(11) **EP 1 196 176 B1**
(45) Date de publication et mention de la délivrance du brevet: **09.06.2004**
(21) Numéro de dépôt: 00958596.9
(22) Date de dépôt: 21.07.2000
(51) Int. Cl.: A61K 31/70, A61K 31/175, A61P 25/00, A61P 25/08

(54) **UTILISATION DE DERIVES DE LA BETA-NAPHTOQUINONE POUR LA FABRICATION DE MEDICAMENTS EXERCANT UN EFFET INHIBITEUR SUR LA LIBERATION DE GLUTAMATE PAR LE CERVEAU**
VERWENDUNG VON BETA-NAPHTAQUINON DERIVATEN ZUR HERSTELLUNG EINES ARZNEIMITTELS MIT EINEM HEMMENDEN EFFEKT AUF DIE GLUTAMATFREISETZUNG DURCH DAS GEHIRN
USE OF BETA-NAPTHOQUINONE DERIVATIVES FOR THE MANUFACTURE OF A MEDICAMENT HAVING AN INHIBITING EFFECT ON THE RELEASE OF GLUTAMATE BY THE BRAIN

(30) Priorité: 21.07.1999 FR 9909469
(43) Date de publication de la demande: 17.04.2002
(73) Titulaire: CENTRE NATIONAL DE LA RECHERCHE SCIENTIFIQUE (CNRS), 75794 Paris Cedex 16 (FR)
(72) Inventeur: ISRAEL, Maurice, F-91440 Bures-sur-Yvette (FR); MOLGO, Jordi, F-92160 Antony (FR); BLOY, Christian, F-69006 Lyon (FR); MATTEI, César, F-75015 Paris (FR)
(74) Mandataire: Peaucelle, Chantal
(86) Numéro de dépôt international: PCT/FR2000/002120
(87) Numéro de publication internationale: WO 2001/005404

(56) Documents cités:
- EP-A- 0 146 338
- EP-A- 0 631 777
- WO-A-98/51291
- US-A- 5 254 683
- MATTEI, C. ET AL: "Naftazone reduces glutamate cerebrospinal fluid levels in rats and glutamate release from mouse cerebellum synaptosomes" NEUROSCI. LETT. (1999), 271(3), 183-186, XP000905383
- AGHA, AZZA M. ET AL: "Lipid peroxidation and lysosomal integrity in different inflammatory models in rats: the effects of indomethacin and naftazone" PHARMACOL. RES. (1995), 32(5), 279-85, 1995, XP000905462
- ZICOT: "Etude multicentrique de l'efficacité et de la tolérance de la naftazone (Mediaven 10mg). Comparaison de deux schemas posologiques" REVUE MEDICALE DE LIEGE, vol. 48, no. 4, avril 1993 (1993-04), pages 224-228, XP000905469
- HERBER, REGINE ET AL: "Reduction and glucuronidation of naftazone by human and rat liver microsomes" DRUG METAB. DISPOS. (1995), 23(12), 1305-14, 1995, XP000905466
- 'The Merck Manual of Diagnosis and Therapy', 1999, MERCK RESEARCH LABORATORIES page 1358

## Description

Nouvelle utilisation de dérivés de la bêta-naphtoquinone et de leurs sels pour la fabrication de médicaments exerçant un effet inhibiteur sur la libération de glutamate dans le cerveau.

L'invention a pour objet une nouvelle utilisation de dérivés de la bêtà-naphtoquinone et de leurs sels pour la fabrication de médicaments exerçant un effet inhibiteur sur la libération de glutamate dans le cerveau.

Le glutamate est le principal neurotransmetteur dans le système nerveux central des mammifères.

Cependant, son accumulation, en excès, dans l'espace extracellulaire du cerveau est toxique pour les neurones, ce qui a amené à considérer qu'il constituait un facteur causal d'un grand nombre de maladies neurologiques, comme l'épilepsie, la maladie de Huntington et dans diverses atteintes liées aux effets délétères dus aux excès de glutamate libéré à la suite d'accidents cérébraux d'origine vasculaire, traumatique ou autre.

L'étude par les inventeurs de certains composés de bêta-naphtoquinone connus comme médicaments vasoprotecteurs a montré qu'ils présentaient, de manière inattendue, des effets inhibiteurs sur la libération de glutamate.

L'invention vise donc une nouvelle utilisation de dérivés de bêta-naphtoquinone pour la fabrication de médicaments à effet inhibiteur sur la libération de glutamate par le cerveau, ces dérives répondant à la formule (I) 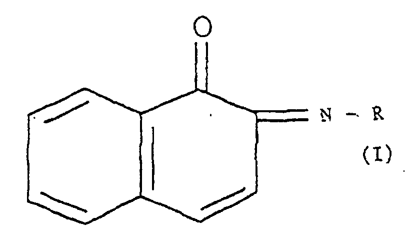 dans laquelle R représente un groupement - NH-CO-NH₂, -NH-CO-CH₃, ou -OH, des dérivés glucuronidés, les composés correspondants, répondant alors à la formule (II) 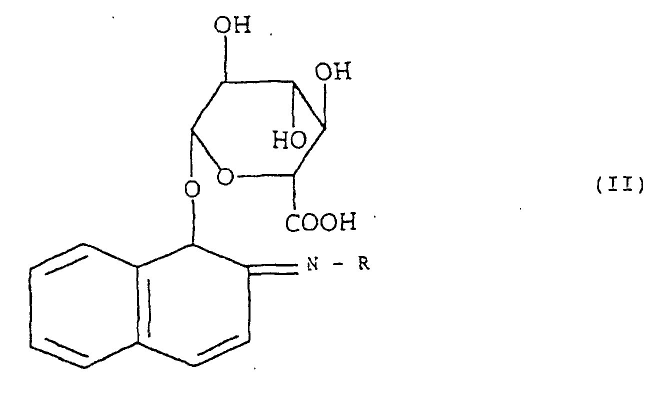 ainsi que de leurs sels d'addition avec des acides. pharmaceutiquement acceptables.

Elle vise tout spécialement l'utilisation de la 2-semicarbazone de la 1,2 naphtoquinone, appelée naftazone selon sa dénomination commune internationale, ainsi que son dérivé glucuronidé correspondant, l'acide 1-(1-hydroxy, 2-naphtyl)semicarbazide-1-β-O-glucopyranosiduronique, de formules, respectivement, (III) et (IV)

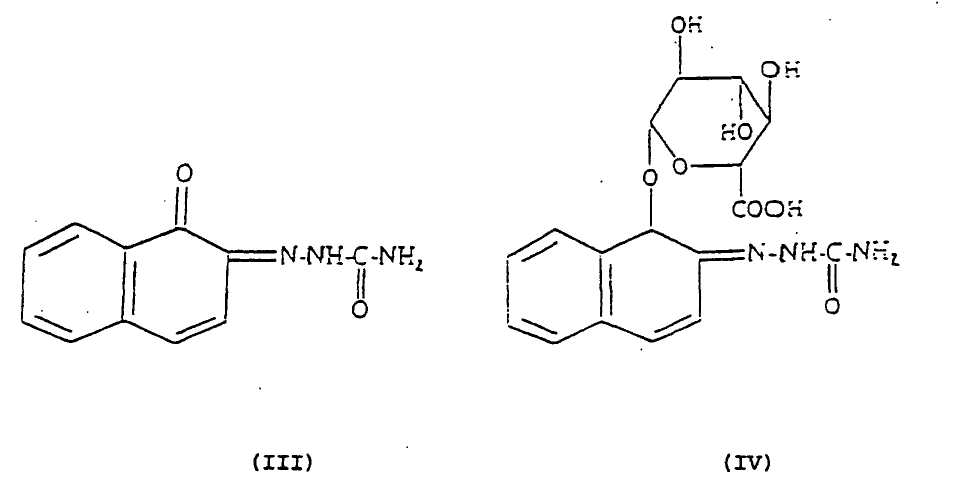

Les sels d'addition avec des acides de ces composés comprennent les sels formés avec des acides minéraux ou des acides organiques.

A titre d'exemple, on citera l'acide chlorhydrique, bromhydrique, sulfurique, phosphorique, ou encore formique, benzoïque, maléique, tartrique, citrique, oxalique, aspartique et des acides alcane sulfoniques.

La préparation des composés utilisés selon l'invention a été largement décrite dans la littérature, par exemple dans le BSM 924 M ou le brevet FR 2103 504.

Les propriétés inhibitrices de ces composés, rapportées ci-après dans les exemples, les rendent particulièrement appropriés pour le traitement de maladies neurologiques et des atteintes liées aux effets délétères du glutamate libéré en excès.

On citera par exemple le traitement de l'épilepsie, de la maladie de Huntington, des effets délétères dus aux excès de glutamate libéré à la suite d'accidents cérébraux d'origine vasculaire, traumatique ou autres.

Ces médicaments sont administrés notamment par voie orale et par voie injectable. Ils se présentent avantageusement sous forme de comprimés, dragées, gélules, capsules, granulés, pour l'administration, par voie orale, ou de solutions ou suspensions pour l'administration par voie injectable.

Les doses seront adaptées selon le patient et la pathologie à traiter et sont par exemple 1 mg à 100 mg/jour.

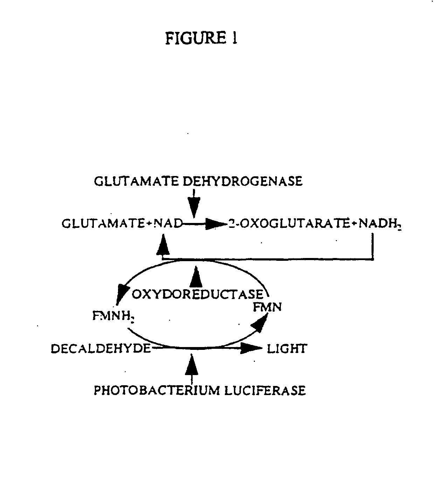

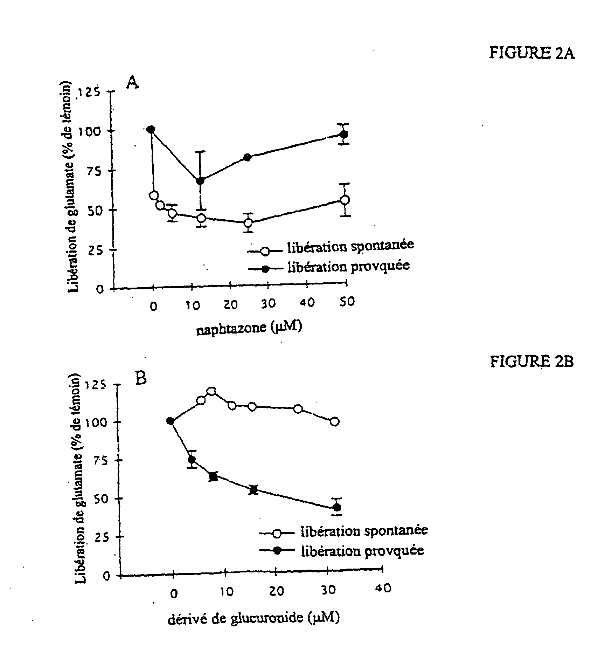

D'autres caractéristiques et avantages de l'invention sont donnés dans les exemples qui suivent dans lesquels il est fait référence aux figures 1 et 2, qui représentent respectivement
- la figure 1, le schéma illustrant le protocole des mesures de chimioluminescence, et
- la figure 2, la libération de glutamate spontanée et celle provoquée en fonction de la concentration en naftazone (fig. 2A) ou en son dérivé glucoronidé (fig. 2B).

### Exemple 1 : Etude des effets inhibiteurs sur le glutamate de la naftazone et de son dérivé glucoronidé.

### A : Etude de l'effet d'un traitement continu avec la naftazone pendant 15 j sur les taux de glutamate dans le CSF de rats normaux

On utilise des rats Sprague-Dawley pesant 200 à 220 g et des souris Swiss-Webster des deux sexes, de 4 à 8 semaines.

On maintient les animaux dans des cages, dans une pièce bien ventilée à 23-24°C, durant un cycle lumière /obscurité de 12h.

Pour étudier les taux de glutamate dans le CSF chez les témoins ou après traitement à la naftazone, on divise les rats mâles en 3 groupes:
- le groupe I (n=8) est utilisé comme témoin. Les rats de ce groupe sont alimentés *per os* pendant 15 jours avec le même véhicule que celui utilisé pour solubiliser la naftazone, à savoir 1% de méthylcellulose, Sigma,
- les animaux des groupes II (n=5) et III (n=5) sont alimentés *per os* pendant 15 jours avec, respectivement, 10 et 100 mg de naftazone par kg, par jour, donné sous forme d'un seul bolus.

On recueille le CSF des rats anesthésiés avec 6% de pentobarbital (i.p.), en opérant selon les protocoles habituels.

Les animaux sont ensuite décapités. Les échantillons de CSF sont centrifugés à 6000g durant 10 min à 10 °C.

On extrait le surnageant et on élimine le culot contenant les dépôts de sang.

Les échantillons sont maintenus dans de l'acide trichloracétique à 2,5%, et conservés à -80°C.

On utilise de l'éther pour éliminer par lavage l'acide trichloracétique des échantillons.

Pour déterminer les taux de glutamate dans le CSF, on procède à des mesures de chimioluminescence selon le protocole décrit dans le schéma donné dans la figure 1. La réaction est basée sur l'oxydation du glutamate en 2-oxoglutarate sous l'action de la glutamate déshydrogénase, qui produit NaDH2, évalué en utilisant la réaction chimioluminescente de photobacterium.

Les échantillons de CSF sont testés. en ajoutant un volume connu d'échantillon au milieu réactionnel qui contient 250 µl de sucrose (120 mM) dans le tampon Tris (120 mM, pH 7,2), 50 µl du mélange enzymatique NAD, FMN NADH-FMN oxydoréductase, luciférase et GDH, et 5 µl de n-décyl aldéhyde.

La lumière émise par la réaction luminescente consécutive à l'oxydation du L-glutamate et à la production de NADH- est détectée par une unité de photomultiplicateur, enregistrée et calibrée par comparaison avec la lumière émise par un standard glutamate.

L'analyse statistique des données est effectuée en utilisant le test *t* de Student pour les échantillons non appariés. Les valeurs sont exprimées en moyenne +/-. SEM. n=nombre d'animaux ou d'expériences effectuées. Les données sont considérées comme significativement différentes des contrôles à *P*<0,05.

Les rats témoins (groupe I) qui ont reçu le véhicule méthylcellulose pendant 15 jours ont une teneur en glutamate dans le CSF allant de 16 à 34 nmol ml⁻¹ avec une valeur moyenne de 22,1 +/- 6,3 nmol ml⁻¹ (n=8).
Le traitement journalier des rats (groupes II et III) pendant 15 jours avec une dose de 10 ou 100 mg/kg de naftazone montre que la teneur en glutamate dans, le CSF des 2 groupes de rats est, respectivement, de 8,1 ± 1,8 (n=5) et 10,8 ± 3,3 nmol ml⁻¹ (n = 5).

Ces résultats montrent que la teneur en glutamate dans le CSF des rats traités avec les 2 doses de naftazone est significativement réduite (*P*=0,001 et *P*=0,004, respectivement), par rapport aux témoins.
De plus, on n'observe pas de différence significative de teneur en glutamate dans le CSF entre les 2 groupes de rats traités avec la naftazone, ce qui indique que l'effet du médicament n'est pas dose-dépendant.

### B : Etude des effets de la naftazone et de son dérivé glucuronide sur la libération de glutamate à partir de synaptosomes de cerveaux de souris

Pour préparer les synaptosomes des fibres moussues, on décapite des souris Swiss- Webster et on enlève rapidement le cervelet.On lave de petits morceaux de tissus (1 à 2 mm³) dans 100 ml d'une solution standard physiologique de mammifère contenant (mM) : NaCl, 136 ; KCl, 5,6 ; MgCl₂ 1,2 ; CaCl₂ 2,2 ; glucose 5,5 ; NaHCO₃ 7,5 ; tampon NaHPO₄ /Na₂HPO₄ 1,2.

On fait passer un courant d'oxygène pendant 10 min.
Pour dissocier les morceaux, on aspire en va-et-vient avec une pipette de 1 ml.

On dilue l'homogéinisat obtenu dans 2 ml d'une solution de Krebs de mammifère et on filtre à travers un tissu en Nylon^{R} (50 µm de maille).

On recueille le filtrat et on laisse sédimenter pendant 30 à 45 min par gravité.

Les synaptosomes dérivés des fibres moussues glutamatergiques sédimentent en raison de leur grande taille avec la fraction nucléaire. Le surnageant est écarté et le culot est remis en suspension dans 1 ml d'une solution standard.

On détecte la libération de glutamate des synaptosomes selon la technique utilisée pour l'évaluer dans le CSF.

Les figures 2A et 2B montrent respectivement les effets de la naftazone (à des concentrations de 0,5 à 50 µM) et de son dérivé glucuronidé, sur la libération spontanée de glutamate (courbe -O-) et sur celle provoquée par dépolarisation (courbe -●-).

Chaque point dans A et B représente la moyenne I.S.E.M. de 3 mesures effectuées en triple. Dans A, le glutamate libéré spontanément est mesuré en continu pendant une exposition de 1 h au médicament testé et comparé à des témoins. La libération de glutamate par dépolarisation est déterminée après une exposition de 1 h au médicament testé et comparée aux témoins.

Les médicaments sont mis à incuber pendant 1h avec des aliquots synaptosomaux avant les mesures.

La libération de glutamate en réponse à la dépolarisation induite par un milieu à forte teneur en K⁺ (30 mM) contenant Ca²⁺ (5mM) n'est pas significativament affectée par la naftazone aux valeurs de concentrations étudiées.

Cependant, comme le montre la figure 2A, la naftazone diminue la libération spontanée de glutamate à partir des synaptosomes. On observe déjà l'effet inhibiteur de la naftazone sur la libération spontanée de glutamate à la plus faible concentration en médicament utilisée ( 0,5 µM). Cet effet est maximal à la concentration de 25 µM.

Des concentrations plus élevées n'apparaissent pas augmenter davantage l'effet inhibiteur.
Lorsqu'on. évalue l'effet du dérivé glucuronidé sur la libération spontanée et sur celle provoquée par K⁺, on constate que le médicament ne réduit pas la libération spontanée de glutamate dans l'intervalle des concentrations utilisées.

Cependant, comme le montre la figure 2B, le dérivé glucuronidé décroît de manière dose-dépendante la libération induite par un milieu à forte teneur en K⁺ (30 mM) contenant Ca²⁺ (5 mM) .

La diminution maximale (environ 60%) est observée à la plus forte concentration en médicament testée (32µM).

### Exemple 2 : Fabrication de compositions pharmaceutiques.

En opérant selon les techniques classiques, on fabrique des comprimés contenant :
- naftazone : 10 mg
- excipient qsp pour 100 mg
   ou des solutés injectables contenant :
- naftazone : 5 mg
- eau stérile qsp : 2 ml.

## Revendications

1. Utilisation de composés dérivés de la bêta-naphtoquinone pour la fabrication de médicaments destinés au traitement de maladies neurologiques et d'atteintes liées aux effets délétères du glutamate libéré en excès, ces composés étant :
(i) soit des composés répondant à la formule (I) 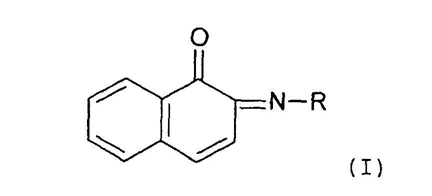 dans laquelle R représente un groupement -NH-CO-NH₂, -NH-CO-CH₃, ou -OH,
(ii) soit des dérivés glucuronidés répondant à la formule (II) 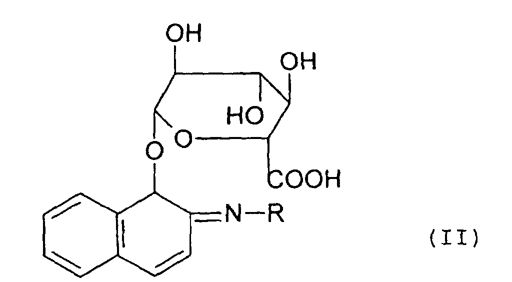
(iii) soit leurs sels d'addition respectifs avec des acides pharmaceutiquement acceptables.

2. Utilisation selon la revendication 1, **caractérisée en ce que** lesdits composés sont soit la 2-semicarbazone de la 1,2 naphtoquinone, son dérivé glucuronidé correspondant, l'acide 1-(1-hydroxy, 2-naphtyl)-semicarbazide-1-β-O-glucopyranosiduroniqué, de formules respectivement, (III) et (IV) 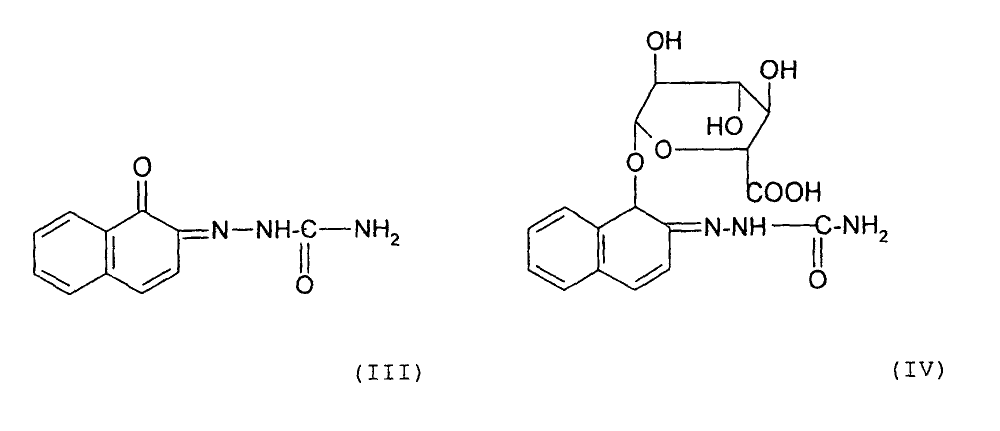 ou leurs sels d'addition respectifs avec des acides pharmaceutiquement acceptables.

3. Utilisation selon la revendication 1 ou 2, **caractérisée en ce que** lesdits sels sont formés avec des acides minéraux ou des acides organiques.

4. Utilisation selon l'une quelconque des revendications 1 à 3, pour la fabrication de médicaments destinés au traitement de l'épilepsie, de la maladie de Huntington, des effets délétères dus aux excès de glutamate libéré à la suite d'accidents cérébraux d'origine vasculaire, traumatique.

5. Utilisation selon l'une quelconque des revendications 1 à 4, **caractérisée en ce que** les médicaments sont administrés par voie orale ou par voie injectable.

## Claims

1. Use of compounds derived from beta-naphthoquinone for the production of medicaments intended for treating neurological diseases and impairments linked to the deleterious effects of excess glutamate release, these compounds being:
(i) either compounds corresponding to formula (I) 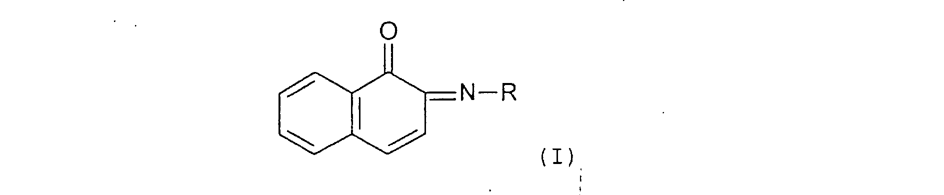 in which R represents an -NH-CO-NH₂, -NH-CO-CH₃, or -OH group,
(ii) or glucuronidated derivatives, corresponding to formula (II) 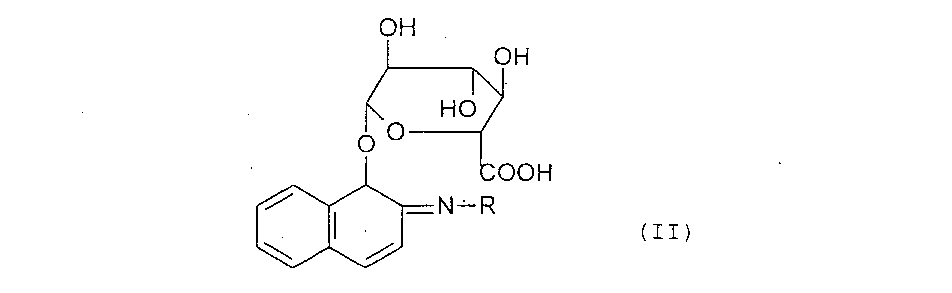
(iii) or their respective addition salts with pharmaceutically acceptable acids.

2. Use according to claim 1, **characterized in that** said compounds are either 1,2 naphthoquinone 2-semicarbazone, its corresponding glucuronidated derivative, 1-(1-hydroxy, 2-naphthyl)semicarbazide-1-β-O-glucopyranosiduronic acid, of formulae (III) and (IV) respectively. 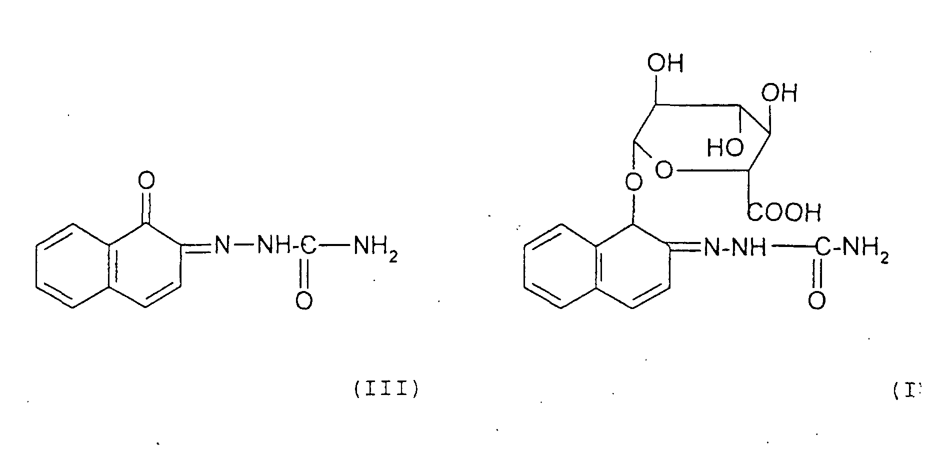 or their respective additions salts with pharmaceutically acceptable acids.

3. Use according to claim 1 or 2, **characterized in that** said salts are formed with mineral acids or organic acids.

4. Use according to any one of claims 1 to 3, for the production of medicaments intended for treating epilepsy, Huntington's disease, deleterious effects due to excess glutamate release following strokes of vascular, traumatic origin.

5. Use according to any one of claims 1 to 4, **characterized in that** the medicaments are administered by oral route or by injectable route.

## Patentansprüche

1. Verwendung von Beta-Naphthochinon-Derivaten zur Herstellung von Arzneimitteln zur Behandlung von neurologischen Krankheiten und Beeinträchtigungen, die mit den nachteiligen Wirkungen von überschüssig freigesetztem Glutamat zusammenhängen, wobei diese Verbindungen sind:
(i) entweder Verbindungen entsprechend der Formel (I) 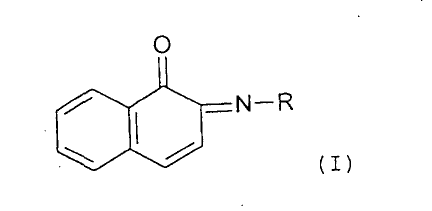 worin R eine Gruppe -NH-CO-NH₂, -NH-CO-CH₃, oder -OH bedeutet,
(ii) oder Glucuronid-Derivate entsprechend der Formel (II) 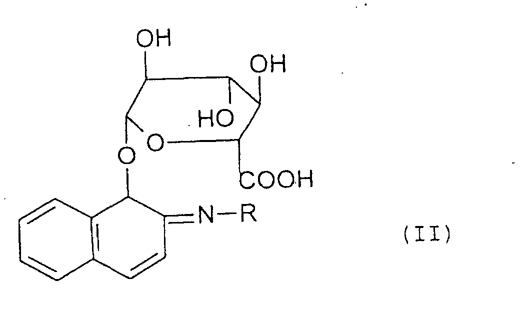
(iii) oder deren jeweilige Additionssalze mit pharmazeutisch annehmbaren Säuren.

2. Verwendung nach Anspruch 1, **dadurch gekennzeichnet, daß** die Verbindungen das 2-Semicarbazon von 1,2-Naphthochinon, dessen entsprechendes Glucuronid-Derivat, die 1-(1-Hydroxy-2-naphthyl)-semicarbazid-1-β-O-glucopyranosiduron-Säure mit den Formeln (III) bzw. (IV) 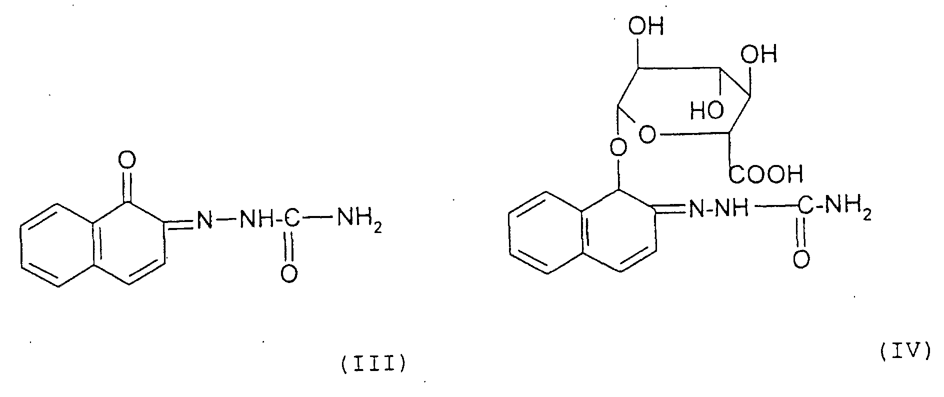 oder deren jeweilige Additionssalze mit pharmazeutisch annehmbaren Säuren sind.

3. Verwendung nach Anspruch 1 oder 2, **dadurch gekennzeichnet, daß** die Salze mit Mineralsäuren oder organischen Säuren gebildet sind.

4. Verwendung nach einem der Ansprüche 1 bis 3 zur Herstellung von Arzneimitteln zur Behandlung von Epilepsie, Huntington-Krankheit, nachteiligen Wirkungen in Folge eines Überschusses von Glutamat, das in Folge von zerebralen Zwischenfällen von vaskulärem oder traumatischem Ursprung freigesetzt ist.

5. Verwendung nach einem der Ansprüche 1 bis 4, **dadurch gekennzeichnet, daß** die Arzneimittel oral oder durch Injektion verabreicht werden.
